# EUROPEAN PATENT SPECIFICATION

(11) **EP 1 899 468 B1**
(45) Date of publication and mention of the grant of the patent: **19.10.2011**
(21) Application number: 06757677.7
(22) Date of filing: 09.05.2006
(51) Int. Cl.: C12N 15/113

(54) **siRNA FOR INHIBITING IL-6 EXPRESSION AND COMPOSITION CONTAINING THEM**
siRNA zur Hemmung der IL-6-Expression und diese enthaltende Zusammensetzung
siRNA pour inhiber l'expression d'IL-6, et composition le contenant

(30) Priority: 20.05.2005 KR 20050042427
(43) Date of publication of application: 19.03.2008
(73) Proprietor: Ajou University Industry-Academic Cooperation Foundation, Suwon-si, Gyeonggi-do 443-749 (KR)
(72) Inventor: BYUN, Hae-Ok, Chungcheongbuk-do 370-803 (KR); CHOI, Bun-Soon 1-301, Jaeseong Villa, Hwaseong-si Gyeonggi-do 445-975 (KR); SOHN, Seong-Hyang, Suwon-si Gyonggi-do 443-733 (KR)
(74) Representative: Grünecker, Kinkeldey, Stockmair & Schwanhäusser
(86) International application number: PCT/KR2006/001737
(87) International publication number: WO 2006/123867

(56) References cited:
- WO-A1-97/38104
- WO-A1-03/070744
- WO-A2-01/68836
- SIOUD M.: 'Induction of inflammatory cytokines and interferon responses by double-stranded and single-stranded siRNAs is sequence-dependent and requires endosomal localization' J. MOL. BIOL. vol. 348, no. 5, 20 May 2005, pages 1079 - 1090, XP004870059

<110> Ajou University Industry cooperation Foundation
<120> siRNA for Inhibiting IL-6 Expression and Composition containing them
<130> EP55697HVpau
<140> not yet assigned
   <141> 2006-05-09
<150> PCT/KR2006/001737
   <151> 2006-05-09
<150> KR10-2005-0042427
   <151> 2005-05-20
<160> 10
<170> KopatentIn 1.71
<210> 1
   <211> 19
   <212> RNA
   <213> Artificial Sequence
<220>
   <223> sense sequence of human IL-6 siRNA
<400> 1
   agacauguaa caagaguaa 19
<210> 2
   <211> 19
   <212> RNA
   <213> Artificial Sequence
<220>
   <223> sense sequence of human IL-6 siRNA
<400> 2
   ggagacuugc cuggugaaa 19
<210> 3
   <211> 19
   <212> RNA
   <213> Artificial sequence
<220>
   <223> sense sequence of human IL-6 siRNA
<400> 3
   ugacaacuca ucucauucu 19
<210> 4
   <211> 19
   <212> RNA
   <213> Artificial sequence
<220>
   <223> anti-sense sequence of human IL-6 siRNA
<400> 4
   uuacucuugu uacaugucu 19
<210> 5
   <211> 19
   <212> RNA
   <213> Artificial Sequence
<220>
   <223> anti-sense sequence of human IL-6 siRNA
<400> 5
   uuucaccagg caagucucc 19
<210> 6
   <211> 19
   <212> RNA
   <213> Artificial sequence
<220>
   <223> anti-sense sequence of human IL-6 siRNA
<400> 6
   agaaugagau gaguuguca 19
<210> 7
   <211> 25
   <212> DNA
   <213> Artificial Sequence
<220>
   <223> sense primer for RT-PCR of Rat PC-12 cell
<400> 7
   tggagtcaca gaaggagtgg ctaag 25
<210> 8
   <211> 25
   <212> DNA
   <213> Artificial sequence
<220>
   <223> anti-sense primer for RT-PCT of Rat PC-12 cell
<400> 8
   tctgaccaca gtgaggaatg tccac 25
<210> 9
   <211> 20
   <212> DNA
   <213> Artificial sequence
<220>
   <223> sense primer for RT-PCR of human neural cell SK-N-MC
<400> 9
   actcacctct tcagaacgaa 20
<210> 10
   <211> 20
   <212> DNA
   <213> Artificial Sequence
<220>
   <223> anti-sense primer for RT-PCR of human neural cell SK-N-MC
<400> 10
   gtctcctcat tgaatccaga 20

## Description

### TECHNICAL FIELD

The present invention relates to an siRNA (small interfering RNA) capable of suppressing interleukin-6 (IL-6) expression and a pharmaceutical composition containing the same.

### BACKGROUND ART

Generally, it was confirmed that IL-6 is abnormally produced in inflammatory diseases, autoimmune diseases and neoplasmic diseases, which has been proposed as pathogenic mechanisms of the diseases (Hirano et al., Immunol. Today, (1990) 11:443-449; Sehgal, P. B., Proc. Soc. Exp. Biol. Med. (1990) 195:183-191; Grau, G. E., Eur. Cytokine Net (1990) 1:203-210; Bauer et al., Ann. Hematol. (1991) 62:203-210; Campbell et al., J. Clin. Invest. (1991) 7:739-742; and Roodman et al., J. Clin. Invest. (1992) 89:46-52). In particular, it was known that IL-6 is associated with a neuropathological process, and its level in blood is increased in the diseases invading the central nervous system. It was found that IL-6 increases a level of tau epitope by stimulating the dementia-associated phosphorylation of the tau-protein in the neuronal cell (Quintanilla RA et al., Exp Cell Res. 2004 Apr 15;295(1):245-257), and the mice lacking IL-6 has an enhanced resistance to glutamate toxicity and an increased viability of the neuronal cell (Fisher J et al., J Neuroimmunol. 2001 Sep 3;119(1):1-9). It was also found that IL-6 amplifies a calcium influx signal for a neurotransmitter NMDA (N-methyl-D-aspartate) through voltage-sensitive calcium channels, which provides a clue that the increased IL-6 level takes an important role in inducing pathological changes of central nervous system diseases in the central nervous system (Qiu Z et al., J Neurosci. 1998 Dec 15;18(24):10445-10456).

More particularly, it has been considered that the abnormal expression of IL-6 is one of the pathogenic mechanisms caused by various diseases including cardiac myxoma; uterine cancer (Kishimoto et al., Ann. Rev. Immunol. 6: 485 (1988)); multiple myeloma; histiocytomas (Taga et al., J. Exp. Med. 166: 967 (1987)); plasmacytoma; hematological diseases including plasma cell dyscrasias, leukemia and lymphoma (Kishimoto, Blood 74: 1 (1989); Taga et al., J. Exp. Med. 166: 967 (1987); and Klein, B. et al., Blood (1991) 78:1198-1204); proliferative glomerulonephritis; activated multiclonal B-cell; types I-IV allergic diseases; rheumatoid arthritis (Hirano et al., Eur. J. Immunol. 18: 1797 (1988)); diabetes (Campbell, I. L. et al., J. Clin. Invest., (1991) 87:739-742); multiple sclerosis; SLE; septic shock; bacterial infections; viral infections; osteoporosis (Roodman, G. D. et al., J. Clin. Invest. (1992) 89:46-52; and Jilka, R. L. et al., Science (1992) 257:88-91); chronic immunodeficiency syndrome and autoimmune immunodeficiency syndrome including AIDS (Med. Immunol. 15: 195-201 (1988)); and inflammatory diseases including inflammatory bowel diseases (Crohn's disease, ulcerative colitis, etc.) (International Patent Publication No. WO1999/47170).

As described above, it has been also known that IL-6 is closely associated with the central nervous system diseases. A high level of the IL-6 was expressed at in cytoplasm and cerebrospinal fluid of patients suffering from multiple sclerosis (Frei et al, J. Neuroimmunol., 31:147(1991)), HTLVI-associated myelopathy and bacterial meningitis, and then the over-expressed IL-6 was detected in the cerebrospinal fluid of the patients suffering from systemic lupus erythematosus and vasculitis-associated central nervous system diseases. It has been known that levels of IL-6, IL-1 and TNF- α were increased in patients infected with HIV-1, indicating these cytokines may play an important role in AIDS nervous system diseases.

Accordingly, there have been various attempts to reduce a level of IL-6 which is considered to be associated in the pathogenic mechanisms of these various diseases. A steroid formulation has been used for suppressing the cytokines in the art, but these medicines may causes severe side-effects such as peptic ulcer if it is administered for an extended period. As a method for regulating a protein expression at a genetic level, a knock out system, which completely removes a function of a desired gene using a homologous recombination technique, has been used until now, but was tedious and so complicated in its process.

In recent years, there have been studied methods using RNA interference (RNAi) as the method for regulating a protein expression at a genetic level. Generally, it was found that a small RNA fragment, named an siRNA (small interfering RNA), has a size of approximately 20 nucleotides, and inhibits a protein expression by specifically binding to mRNA complementary to the small RNA fragment. It was confirmed that, when double-strand RNA is introduced into nematode, the double-strand RNA induces gene silencing by binding to mRNA in the cell in a sequence-specific manner, which is, therefore, referred to as RNA interference. It was known that it was determined according to a complementarity between mRNA and its corresponding 21-25 nucleotide RNA whether degradation or transcriptional suppression of mRNA having its complementary base sequence is induced by the siRNA. Therefore, it was revealed that the mRNA was degraded if the complementarity is 100 %, and the mRNA was transcriptionally suppressed if the complementarity ranges from 80 to 90 %. It has been known that the siRNA provides an important basic knowledge in developing an effective RNAi technique by rapidly and effectively suppressing gene expression in eukaryotic cells.

In the above-mentioned methods, it was, however, not considered that the gene expression may not be suppressed completely, as well as that a suppression effect of the gene is insignificant in targeting a gene whose mRNA or protein is produced at a large amount and a gene that encodes a very stable protein, and the RNA interference effect may not be exhibited at all between the mRNA and the nucleotides having its complementary base sequence. That is to say, in order to prepare siRNA, a target sequence having approximately 19-20 nucleotides, complementary to the siRNA, was determined from a base sequence of the gene that may induce its down regulation. Also, it was revealed that a theoretically deduced siRNA does not always suppress an ability of the targeted mRNA in an effective manner. There has been required a method for preparing siRNA having multiple target sites in one gene, followed by directly determining an RNA interference effect of the siRNA using a cell or animal model.

### DISCLOSURE OF INVENTION

The present invention is designed to solve the problems of the prior art, and therefore it is an object of the present invention to provide an siRNA (small interfering RNA) capable of lowering an IL-6 level, more particularly of suppressing interleukin-6 (IL-6) expression so as to treat the various diseases as described above.

It is also another object of the present invention to provide a pharmaceutical composition containing the siRNA or mixtures thereof.

Also, it is still another object of the present invention to provide a novel use of the pharmaceutical composition containing the siRNA or mixtures thereof in order to treat the inflammatory diseases, the autoimmune diseases, the neoplasmic disease and the central nervous system diseases as described above.

In order to accomplish the above object, the present invention provides a double-strand siRNA (small interfering RNA) selected from the group consisting of SEQ ID NO: 1 and its complementary sequence SEQ ID NO: 4, SEQ ID NO: 2 and its complementary sequence SEQ ID NO: 5, and SEQ ID NO: 3 and its complementary sequence SEQ ID NO: 6, and the present invention also provides a pharmaceutical composition for suppressing IL-6 expression, wherein the pharmaceutical composition contains the siRNA or mixtures thereof.

Also, the present invention provides a pharmaceutical composition for treating a disease selected from the group consisting of inflammatory diseases, autoimmune diseases, neoplasmic disease or central nervous system diseases, wherein the pharmaceutical composition contains the siRNA or mixtures thereof. The present invention also a pharmaceutical composition, wherein the central nervous system diseases is selected from the group consisting of Alzheimer's disease, hypochondria, epilepsy, migraine, multiple sclerosis, pains, Pakinson's disease and schizophrenia.

That is to say, the present invention provides an siRNA having a specific base sequence capable of treating the diseases by suppressing IL-6 expression to lower an IL-6 level in blood, a pharmaceutical composition containing the siRNA, and a use for treating a disease selected from the group consisting of inflammatory diseases, autoimmune diseases, neoplasmic disease or central nervous system diseases using the same.

The inventors has unexpectedly found that the present invention may be accomplished by the double-strand siRNA selected from the group consisting of SEQ ID NO: 1 and SEQ ID NO: 4, SEQ ID NO: 2 and SEQ ID NO: 5, and SEQ ID NO: 3 and SEQ ID NO: 6 as described later, based on the fact that the double-strand RNA having the specific base sequence may control or suppress IL-6 expression using the RNA interference effect.

Hereinafter, the siRNA for suppressing IL-6 expression according to the present invention and the pharmaceutical composition containing the same will be described in detail.

The present invention provides a double-strand siRNA selected from the group consisting of SEQ ID NO: 1 and its complementary sequence SEQ ID NO: 4, SEQ ID NO: 2 and its complementary sequence SEQ ID NO: 5, and SEQ ID NO: 3 and its complementary sequence SEQ ID NO: 6.

The methods for producing siRNA having the above-mentioned base sequence includes, but is not limited to, a method for directly synthesizing siRNA in a chemical manner (Sui G et al., (2002) A DNA vector-based RNAi technology to suppress gene expression in mammalian cells. Proc Natl Acad Sci USA 99: 5515-5520), a method for synthesizing siRNA using an *in vitro* transcription (Brummelkamp TR et al., (2002) A system for stable expression of short interfering RNAs in mammalian cells. Science 296: 550-553), a method in which a long double-strand RNA synthesized by an *in vitro* transcription is digested with enzymes such as RNaseIII family enzymes (Paul CP et al., (2002) Effective expression of small interfering RNA in human cells. Nature Biotechnology 20: 505-508), an expression method using the introduction of an siRNA expression plasmid or a viral vector into cells (Lee NS et al., (2002) Expression of small interfering RNAs targeted against HIV-1 rev transcripts in human cells. Nature Biotechnology 20: 500-505), and an expression method using the introduction of a PCR (polymerase chain reaction)-derived siRNA expression cassette into cells (Castanotto D et al., (2002) Functional siRNA expression from transfected PCR products. RNA 8: 1454-1460), etc.

The present invention also provides a recombinant vector containing at least one siRNA according to the present invention. For example, various expression systems such as chromosomes, episomes and induced viruses may be used herein. More specifically, the recombinant vector, which may be used herein, includes, but is not limited to, bacterial plasmids, transposons, yeast episomes, insertion sequences, yeast chromosome sequences, and viruses derived from baculovirus, papllioma viruses such as SV40, vaccine virus, adenovirus, avipox virus, pseudorabies virus and retrovirus. These recombinant vectors may be cosmid or phagemid derivatives.

Each of the siRNA sequences may be, for example, inserted into a recombinant expression vector according to the known methods as apparent to those skilled in the art pertaining to the present invention, as described in the disclosure (Molecular cloning: A laboratory manual, Sambrook et al., 2nd Ed., Cold Spring Harbor Laboratory Press, Cold Spring Harbor, N.Y., 2001).

The recombinant vector may include a base sequence controlling the siRNA expression of the present invention, and the base sequence may be selected according to the used host cells.

The present invention also provides a host cell containing the recombinant vector according to the present invention. Introduction of the host cell into the recombinant vector may be carried out according to the methods as apparent to those skilled in the art pertaining to the present invention, including, but is not limited to, a transformation using calcium phosphate (Graham, F.L. et al., Virology, 52:456(1973)), a transformation using DEAE dextran, a transformation using microinjection (Capecchi, M.R., Cell, 22:479(1980)), a transformation using a cationic lipid (Wong, T.K. et al., Gene, 10:87(1980)), an electroporation (Neumann E. et al., EMBO J., 1:841(1982)), an a transformation or transfection (Basic methods in molecular biology, Davis et al., 1986 and Molecular cloning: A laboratory manual, Davis et al., 1986).

For example, the host cell includes, but is not limited to, bacterial cells such as *streptococci, staphylococci, E. coli* and *Bacillus subtilis,* yeast cells, and fungal cells such as *Aspergillus* and *Streptomyces,* insect cells such as *Drosophilia*) S2 and *Spodoptera* Sf9, and mammalian or plant cells to be treated.

The present invention also provides a pharmaceutical composition containing the double-strand siRNA or mixtures thereof. The pharmaceutical composition is prepared by mixing the siRNA with a pharmaceutically available carrier uniformly or homogeneously. The preferred liquid carrier includes, but is not limited to, water, saline and a pharmaceutically available buffer, and the preferred non-liquid carrier includes, but is not limited to, a mineral oil, a neutral oil and mixtures thereof.

The pharmaceutical composition of the present invention is, for example, in a solid state, a suspended state, or a liquid state, and is preferably an injectable suspending or liquid formulation, or an injectable powder formulation which may be used after being dissolved in a suitable solvent before administration. These formulations may be prepared according to the methods known to those skilled in the art pertaining to the present invention.

Also, the pharmaceutical composition of the present invention may be included in a suitable amount for the purpose of stabilization of active components, osmoregulation, and pH adjustment, etc. The additive includes, but is not limited to, a stabilizer, an osmoregulating agent, a pH controller, a wetting agent, an emulsifier, a dispersing agent, antiseptic, etc.

Routes of administration of the pharmaceutical composition of the present invention include, but are not limited to, oral, topical, subcutaneous, transdermal, subdermal, intramuscular, intraperitoneal, intravesical, intraarticular, intraarterial, intravenous, intradermal, intracranial, intralesional, intraocular, intrapulmonary, intrathecal and intraprostate. Also, administration of the pharmaceutical composition of the present invention includes, but are not limited to, nasal inhalation, pulmonary inhalation, impression into skin and electrocorporation.

Depending on the routes of administration, the volume per dose is preferably about 0.001 to 100 ml per dose, more preferably about 0.01 to 50 ml per dose and most preferably about 0.1 to 30 ml per dose. The siRNA administered per dose can be preferably administered at an amount of about 1 ng to 100 mg/kg, more preferably an amount of about 10 ng to 1 mg/kg, and most preferably an amount of about 100 ng to 100 µg/kg.

The administered dosage of the pharmaceutical composition according to the present invention can be varied in a suitable dose according to factors such as formulation methods, administration methods, age, body weight and sex of the patient, severity of the disease, food, administration duration, administered passage, excretion rate and reactive sensitivity.

### BRIEF DESCRIPTION OF THE DRAWINGS

These and other features, aspects, and advantages of preferred embodiments of the present invention will be more fully described in the following detailed description, taken accompanying drawings. In the drawings:

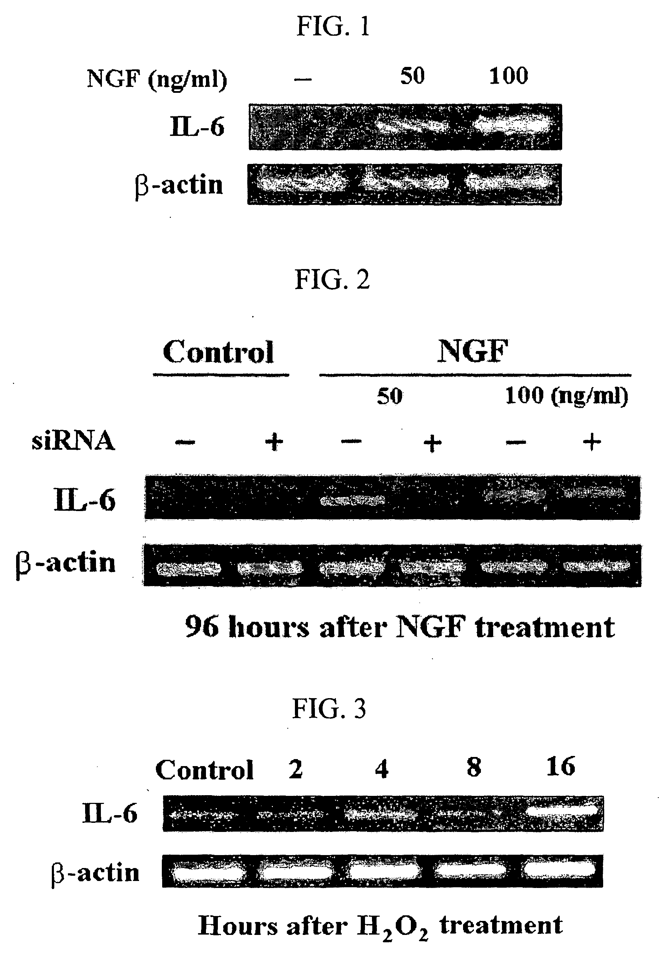

FIG. 1 is a RT-PCR result showing that an IL-6 mRNA level is increased in a murine PC-12 cell treated with NGF (a nerve growth factor).
FIG. 2 is a RT-PCR result showing IL-6 mRNA levels in (+) group and (-) group, wherein the (+) and (-) groups are referred to as groups in which the siRNA mixture according to the present invention is administered or not administered into a control in which the murine PC-12 cell is not treated with NGF, and an experimental group in which the murine PC-12 cell is treated with NGF, respectively.
FIG. 3 is a RT-PCR result showing that an IL-6 RNA level is varied in a human neuronal cell SK-N-MC treated with hydrogen peroxide, depending on the passage of time.

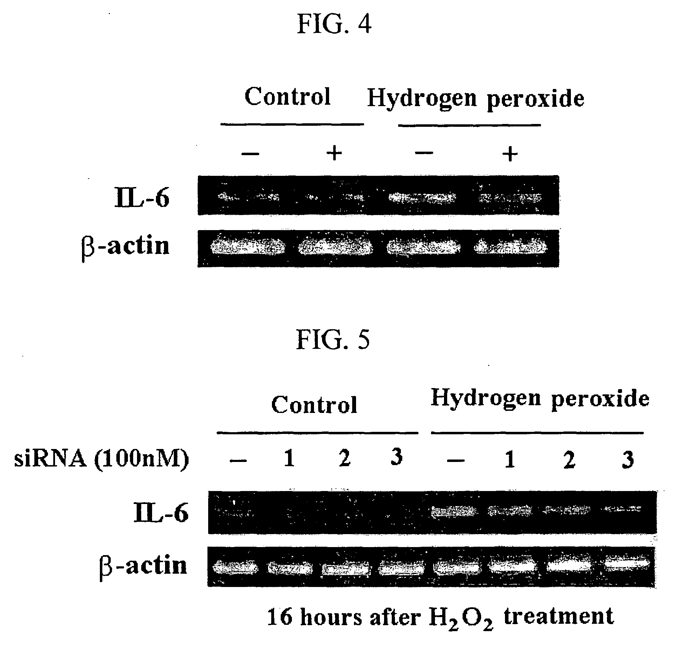

FIG. 4 is a RT-PCR result showing IL-6 mRNA levels in (+) group and (-) group, wherein the (+) and (-) groups are referred to as groups in which the siRNA mixture according to the present invention is administered or not administered into a control in which the human neuronal cell SK-N-MC is not treated with NGF, and an experimental group in which the human neuronal cell SK-N-MC is treated with NGF, respectively.
FIG. 5 is a RT-PCR result showing IL-6 mRNA levels in (+) group and (-) group, wherein the (+) and (-) groups are referred to as groups in which the siRNA according to the present invention is administered or not administered into a control in which the human neuronal cell SK-N-MC is not treated with NGF, and an experimental group in which the human neuronal cell SK-N-MC is treated with NGF, respectively. In FIG. 5, Lane "1" shows a result obtained by administering the double-strand siRNA composed of SEQ ID NO: 1 and its complementary sequence SEQ ID NO: 4; Lane "2" shows a result obtained by administering the double-strand siRNA composed of SEQ ID NO: 2 and its complementary sequence SEQ ID NO: 5; and Lane "3" shows a result obtained by administering the double-strand siRNA composed of SEQ ID NO: 3 and its complementary sequence SEQ ID NO: 6.

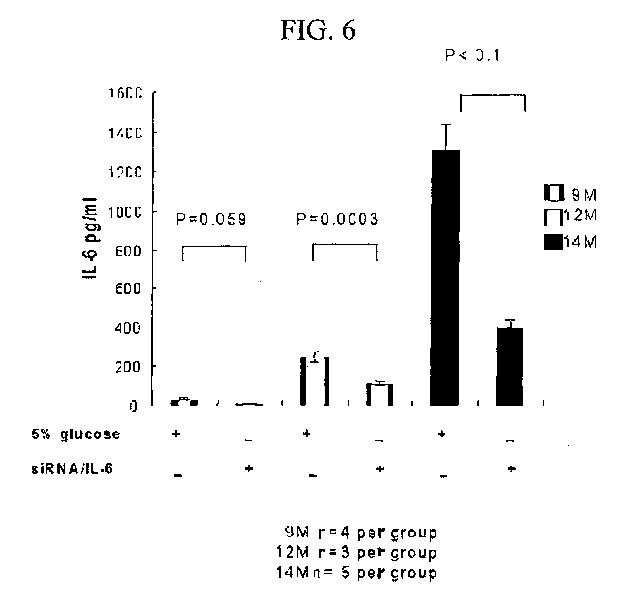

FIG. 6 shows a result showing IL-6 mRNA levels in serum after the siRNA is administered into 9-month, 12-month, 14-month normal mice (from right to left), respectively, using an ELISA system. As shown in FIG. 6, it was revealed that a concentration of the IL-6 mRNA in blood is increased as a mouse gets older, and the increased concentration of the IL-6 mRNA in blood is more decreased when the IL-6 siRNA is administered at a dose of 1.5 ug/mouse than when the control, glucose, is administered into the mouse. As a result, it might be confirmed that the siRNA sequence, as described herein, is effective *in vivo* in a mouse model.

### BEST MODES FOR CARRYING OUT THE INVENTION

Hereinafter, preferred embodiments of the present invention will be described in detail with reference to the accompanying drawings.

### Example 1: Preparation of siRNA

Double-strand siRNAs were chemically synthesized, the double-strand siRNAs being selected from the group consisting of SEQ ID NO: 1 and its complementary sequence SEQ ID NO: 4, SEQ ID NO: 2 and its complementary sequence SEQ ID NO: 5, and SEQ ID NO: 3 and its complementary sequence SEQ ID NO: 6. The double-strand siRNAs were synthesized by Dharmacon, Inc. (Lafayette, CO).

### Example 2: Measurement of Effect of siRNA using Murine PC-12 Cell

16 hours before transfection, 2x10⁵ cells/ml of murine PC-12 cells were spread on a 35 mm dish. A murine PC-12 cell line was transfected by treating the murine PC-12 cell line with 3 µℓ of the transfection reagent oligofectamin^{™} (Invitrogen) and 180 mM siRNA prepared by mixing the three pairs of the double-strand siRNAs of the present invention in an equivalent amount. Four hours after transfection, some of the samples were treated with 50 or 100 ng/mℓ of a nerve growth factor (NGF)(BD Bioscience, Bedford, MA) to stimulate IL-6 expression, incubated for 96 hours, and then an IL-6 mRNA level was measured using a RT-PCR method as described later.

FIG. 1 shows a result that an IL-6 expression is increased in the murine PC-12 cell which is not treated with the siRNA but with only NGF, and FIG. 2 shows a result that the siRNA inhibits the IL-6 mRNA level increased by the NGF.

As shown in FIG. 2, it was revealed that the IL-6 mRNA level is decreased in the experimental group which is treated with the siRNA, followed by with the NGF. However, as a dose of the NGF was increasing, that is to say if the murine PC-12 cell was treated with 100 ng/mℓ of the NGF, the suppression effect by the siRNA is relatively lower than if the murine PC-12 cell was treated with 50 ng/mℓ of the NGF.

### Example 3: Measurement of Effect of siRNA using Human Neuronal Cell SK-N-MC

16 hours before transfection, 2x10⁵ cells/ml of human neuronal cells were spread on a 35 mm dish. Each of the human neuronal cells was pre-treated with 100 nM of the each siRNA of the present invention or pre-treated with 180 nM of the siRNA mixture prepared by mixing the three pairs of the double-strand siRNAs of the present invention in an equivalent amount. Then, the human neuronal cells were transfected by treating the human neuronal cells with 3 µℓ of the transfection reagent oligofectamin^{™}. 16 hours after transfection, the human neuronal cells were treated with 200 µ M hydrogen peroxide to stimulate IL-6 expression. 2, 4, 8 and 16 hours after treatment of the hydrogen peroxide, the human neuronal cells were washed with a DMEM-free medium, and then an IL-6 mRNA level was measured using a RT-PCR method as described later.

FIG. 3 shows a result that an IL-6 expression is increased in the human neuronal cell SK-N-MC which is not treated with the siRNA but with only hydrogen peroxide, and FIGs. 4 and 5 show results that the siRNA mixture and the each siRNA inhibits the IL-6 mRNA level increased by the hydrogen peroxide, respectively.

As shown in FIG. 3, it was revealed that the IL-6 mRNA level is increased when the human neuronal cell SK-N-MC is treated with the hydrogen peroxide. As shown in FIGs. 4 and 5, it was revealed that the increased IL-6 mRNA level is decreased by treating the cell with the siRNA of the present invention, and the suppression effect of the IL-6 mRNA by the double-strand siRNA, which is composed of SEQ ID NO: 3 and its complementary sequence SEQ ID NO: 6, is relatively excellent when compared to the two other pairs of the siRNAs.

### Example 4: RT-PCR for Analyzing IL-6 mRNA Level

500 µℓ of trizol was added to every 35 mm dish, pipetted, and then transferred into a 2 mℓ tube and stirred vigorously. Chloroform was added 0.2 volume of the trizol, and then the mixture was stirred vigorously and kept in ice for 5 minutes. Subsequently, the mixture was centrifuged at 4 °C for 15 minutes in a rotary speed of 12,000 rpm. A RNA fraction (an upper transparent layer) was transferred into a new tube, an equivalent amount of isopropanol was added to the tube, and then the tube was shaken up and down and kept in ice for 15 minutes. Subsequently, the mixture was centrifuged at 4 °C for 15 minutes in a rotary speed of 12,000 rpm, and then a supernatant was removed off and the remaining RNA pellet was dissolved in 500 µℓ of 80 % ethanol prepared using a RNase-free purified water. The resultant RNA solution was shaken up and down, and then centrifuged for 5 minutes in a rotary speed of 12,000 rpm and a supernatant was removed off. The tube in which the RNA was precipitated was centrifuged under a vacuum environment to dry the RNA pellet, and 10-15 µℓ of RNase-free water was added to the tube, and then incubated at 65 °C for 15 minutes in a heating block while tapping repeatedly. When the heating was completed, the tube was centrifuged to precipitate a solution containing RNA, and stored in ice.

The solution containing RNA was quantitified to conduct a RT-PCR reaction. At this time, SuperScript^{™} First-Strand Synthesis System (Invitrogen) were used as all reagents of the RT-PCR reaction, and the RT-PCR reaction was carried out according to a manufacturer's manual. A sense primer of SEQ ID NO: 7 and an anti-sense primer of SEQ ID NO: 8 were used in the RT-PCR reaction of the murine PC-12 cell, and a sense primer of SEQ ID NO: 9 and an anti-sense primer of SEQ ID NO: 10 were used in the RT-PCR reaction of the human neuronal cell SK-N-MC.

### Example 5: Experiment on In vivo Murine siRNA Injection

The IL-6 siRNA, prepared in Example 1 of the present invention, was mixed with a transfection reagent jetPEI (Polyplus transfection, San Marcos, CA), and the resultant mixture was administered into the intra-tail vein. The injection was carried out 4 times at an interval of 3 days with 1.5 µg of the mixture/mouse, and an IL-6 level in serum was analyzed 2 days after the final injection, using an ELISA system.

### INDUSTRIAL APPLICABILITY

As described above, the siRNA having a specific base sequence according to the present invention can suppress the IL-6 expression by degrading or suppressing IL-6 mRNA. Accordingly, the siRNA of the present invention and the pharmaceutical composition containing the same may be useful to treat inflammatory diseases, autoimmune diseases, neoplasmic disease and central nervous system diseases, which are all caused by the over-expressed IL-6 level, by lowering an IL-6 level.
## Claims

1. A double-strand siRNA(small interfering RNA) selected from the group consisting of SEQ ID NO: 1 and its complementary sequence SEQ ID NO: 4, SEQ ID NO: 2 and its complementary sequence SEQ ID NO: 5, and SEQ ID NO: 3 and its complementary sequence SEQ ID NO: 6.

2. A pharmaceutical composition for suppressing interleukin-6 (IL-6) expression, wherein the pharmaceutical composition contains the siRNA as defined in claim 1, and mixtures thereof.

3. A pharmaceutical composition for treating a disease selected from the group consisting of inflammatory diseases, autoimmune diseases, neoplasmic disease or central nervous system diseases, wherein the pharmaceutical composition contains the siRNA as defined in claim 1, and mixtures thereof.

4. The pharmaceutical composition according to claim 3,
wherein the central nervous system diseases is selected from the group consisting of Alzheimer's disease, hypochondria, epilepsy, migraine, multiple sclerosis, pains, Pakinson's disease and schizophrenia.

## Patentansprüche

1. Doppelsträngige siRNA ("*small interfering RNA*") ausgewählt aus der Gruppe bestehend aus SEQ ID NR: 1 und ihrer komplementären Sequenz SEI ID NR: 4, SEQ ID NR: 2 und ihrer komplementären Sequenz SEQ ID NR: 5, und SEQ ID NR: 3 und ihrer komplementären Sequenz SEQ ID NR: 6.

2. Pharmazeutische Zusammensetzung zum Supprimieren der Interleukin-6 (IL-6)-Expression, wobei die pharmazeutische Zusammensetzung die siRNA, wie in Anspruch 1 definiert, und Mischungen davon enthält.

3. Pharmazeutische Zusammensetzung zum Behandeln einer Krankheit, die ausgewählt ist aus der Gruppe bestehend aus: Entzündungskrankheiten, Autoimmunerkrankungen, neoplasmische Erkrankung oder Erkrankung des zentralen Nervensystems, wobei die pharmazeutische Zusammensetzung die siRNA, wie in Anspruch 1 definiert, und Mischungen davon enthält.

4. Pharmazeutische Zusammensetzung nach Anspruch 3, wobei die Erkrankung des zentralen Nervensystems ausgewählt ist aus der Gruppe bestehend aus: Alzheimer Krankheit, Hypochondrie, Epilepsie, Migräne, Multiple Sklerose, Schmerzen, Parkinson Krankheit und Schizophrenie.

## Revendications

1. ARNsi (petit ARN interférent) double brin choisi parmi le groupe constitué de la SEQ ID N° : 1 et sa séquence complémentaire SEQ ID N° : 4, de la SEQ ID N° : 2 et sa séquence complémentaire SEQ ID N° : 5, et de la SEQ ID N° : 3 et sa séquence complémentaire SEQ ID N° : 6.

2. Composition pharmaceutique destinée à supprimer l'expression de l'interleukine-6 (IL-6), la composition pharmaceutique contenant l'ARNsi tel que défini dans la revendication 1, et des mélanges de celui-ci.

3. Composition pharmaceutique destinée à traiter une maladie choisie parmi le groupe constitué de maladies inflammatoires, de maladies auto-immunes, de maladies néoplasmiques ou de maladies du système nerveux central, la composition pharmaceutique contenant l'ARNsi tel que défini dans la revendication 1, et des mélanges de celui-ci.

4. Composition pharmaceutique selon la revendication 3, dans laquelle les maladies du système nerveux central sont choisies parmi le groupe constitué de la maladie d'Alzheimer, de l'hypocondrie, de l'épilepsie, de la migraine, de la sclérose en plaques, de douleurs, de la maladie de Parkinson et de la schizophrénie.
